# EUROPÄISCHE PATENTSCHRIFT

(11) **EP 2 254 525 B1**
(45) Veröffentlichungstag und Bekanntmachung des Hinweises auf die Patenterteilung: **21.09.2016**
(21) Anmeldenummer: 09713092.6
(22) Anmeldetag: 05.02.2009
(51) Int. Cl.: A61F 2/60, A61F 2/30, A61F 2/76, G01L 5/22

(54) **ROHRFÖRMIGE SENSOREINRICHTUNG**
TUBULAR SENSOR DEVICE
DISPOSITIF DE DÉTECTION TUBULAIRE

(30) Priorität: 21.02.2008 DE 102008010281
(43) Veröffentlichungstag der Anmeldung: 01.12.2010
(73) Patentinhaber: Otto Bock Healthcare Products GmbH, 1070 Wien (AT)
(72) Erfinder: SCHULZE, Werner, 37083 Göttingen (DE); WALD, Michael, 31139 Hildesheim (DE)
(74) Vertreter: Stornebel, Kai
(86) Internationale Anmeldenummer: PCT/EP2009/000778
(87) Internationale Veröffentlichungsnummer: WO 2009/103423

(56) Entgegenhaltungen:
- EP-A1- 1 559 384
- EP-A2- 1 143 230
- DE-A1- 3 405 168
- DE-A1- 3 528 364
- DE-A1-102005 051 495
- US-A- 4 932 253
- US-A1- 2003 094 081
- US-A1- 2007 255 424

## Beschreibung

### Gebiet der Erfindung

Die Erfindung betrifft eine Sensoreinrichtung zur Messung einer axialen Kraft in Rohrlängsrichtung eines Rohres und/oder mindestens eines Drehmomentes mit einem Messelement, das einen über ein Rohransatzstück mit dem Rohr verbundenen Adapteranschluss aufweist, über den das Rohr an einen Justieradapter fest ankoppelbar ist, wobei die axiale Kraft und das Drehmoment über das Messelement in den Justieradapter einleitbar und mittels Dehnmessstreifen messbar sind, die in einem Sensorbereich zwischen dem Adapteranschluss und einem dem Adapteranschluss abgewandten Ende des Rohransatzstückes an DMS-Messstellen angeordnet sind.

Solche Sensoreinrichtungen können beispielsweise bei Rohrkonstruktionen allgemein oder speziell bei Handhabungseinrichtungen bzw. Industrierobotern oder für Prothesen eingesetzt werden.

So ist beispielsweise von der Firma Otto Bock Healthcare GmbH in Duderstadt eine Sensoreinrichtung zur Messung eines Drehmomentes quer zur Rohrlängsrichtung des Rohres bekannt. Dabei dient das Rohr zur Verbindung zwischen einem steuerbaren künstlichen Kniegelenk und einem Prothesenfuß. Das Rohr ist über eine am Kniegelenk angeordnete Klemmschelle mit dem Kniegelenk verbindbar. Der Prothesenfuß weist dabei einen so genannten Justieradapter auf, an den das Rohr über einen mit ihm verbundenen Adapteranschluss über Justierschrauben fest ankoppelbar und in gewissen Grenzen justierbar ist. Der Adapteranschluss weist ein Rohransatzstück auf, das an den Adapteranschluss angeformt ist, wobei das Rohr mit seinem dem Adapteranschluss zugewandten Ende über das Rohransatzstück geschoben und mit ihm verklebt ist. Das Rohransatzstück und der Adapteranschluss bilden dabei ein Messelement über das die axiale Kraft und das Drehmoment in den Justieradapter einleitbar und mittels am Rohransatzstück angeordneten Dehnmessstreifen messbar sind.

Das bekannte Rohransatzstück besteht im Prinzip aus einem an den Adapteranschluss angeformten zylinderförmigen Ansatz, der quer zu seiner Längsachse einen Durchbruch aufweist und der an seiner dem Adapteranschluss abgewandten Stirnseite über zwei angeformte einander gegenüberliegende stegförmige Balken mit der Bodenfläche eines topfförmigen Endes des Ansatzstückes verbunden ist. Die beiden Stege bilden einen Sensorbereich mit jeweils einer Messstelle mit vier in einer Brückenschaltung angeordneten Dehnmessstreifen. Sowohl der zylinderförmige Ansatz als auch der topfförmige Ansatz sind an ihrer Mantelfläche mit der Rohrwandung des Rohres verklebt.

Nachteilig dabei ist, dass zum einen das Messelement mit seinem Rohransatzstück relativ kompliziert und damit kostenintensiv aufgebaut ist und dass zum anderen die Abgrenzung der Axialkraft gegenüber den Störlasten und den zu messenden Drehmomenten problematisch ist.

Weiterhin ist aus der EP 1 559 384 B1 eine Sensoreinrichtung bzw. ein Drehmomentsensor für die Messung eines Drehmoments bekannt, der als eine virtuelle Drehachse außerhalb des Sensors bildende Sensorstruktur aufgebaut ist und der unmittelbar unterhalb des Kniegelenks angeordnet ist. Dabei weist der Drehmomentsensor bzw. das Messelement eine Mehrgelenkstruktur mit elastisch verformbaren Gelenken auf.

Nachteilig dabei ist, dass das Messelement relativ aufwendig ausgebildet ist und Bauraum beansprucht, der von wesentlichen Funktionselementen bereits besetzt sein kann.

Weiterhin ist aus der DE 10 2005 051 495 A1 eine Sensoranordnung für die Messung von Kräften und Momenten bekannt, bei der das Messelement als Teil eines Justieradapters ausgebildet ist.

Nachteilig dabei ist, dass der Justieradapter relativ voluminös und aufwendig ausgebildet ist.

Die US 2007/0255424 A1 betrifft eine Sensoreinrichtung für eine prothetische Einrichtung mit einem Trägerteil, an dessen Außenseite Dehnmessstreifen angebracht sind. An einem ersten Ende sind Einstellschrauben zum Befestigen einer Protheseneinrichtung angeordnet, das zweite Ende ist zum Einklemmen auf einen Pylonenbereich ausgebildet.

Die US 4,932,253 betrifft eine an einem Rohr befestigte Kraftmesszelle, die an einem polierten Rohr zwischen einer Rohrklemme und einer Hängerstange einer hin- und hergehenden Pumpe angeordnet ist. Die Kraftmesszelle dient ausschließlich zur Erfassung axial wirkender, schwellender Lasten.

Die US 2003/0094081 A1 betrifft einen elektronischen Drehmomentschlüssel, mit einem zylindrischen Grundkörper, der in einer Hülse angeordnet ist. An dem zylindrischen Grundkörper ist in einem Bereich mit einem verlängerten Durchmesser ein Messbereich ausgebildet, in dem vier Dehnmessstreifen in einer Brückenschaltung angeordnet sind über die Dehnmessstreifen wird das auf den Werkzeugkopf übertragene Drehmoment gemessen.

### Aufgabenstellung

Aufgabe der vorliegenden Erfindung ist es daher, einen Messsensor zur Verfügung zu stellen, der einfach und kostengünstig zu fertigen ist und der eine reproduzierbare, sichere und schnelle Ermittlung sowohl von Axialkräften als auch von Drehmomenten ermöglicht.

### Darstellung der Erfindung

Diese Aufgabe wird in Verbindung mit dem Oberbegriff des Anspruchs 1 dadurch gelöst, dass auf dem Umfang des Sensorbereiches mindestens drei DMS-Stellen angeordnet sind und zusätzliche DMS-Stellen auf dem Umfang des Sensorbereiches zur getrennten Ermittlung des Drehmomentes und der axialen Kraft angeordnet sind.

Durch die rohrförmige Ausbildung des Rohransatzstückes und des Sensorbereiches kann auf gewichtsreduzierende Durchbrüche quer zur Längsrichtung verzichtet werden. Dadurch, dass der Sensorbereich sich auf die gesamte Länge des Rohansatzstückes zwischen Adapteranschluss und dem dem Adapteranschluss abgewandten Ende des Rohransatzstückes erstreckt und in seinem Außendurchmesser geringer ist als das Ende des Rohransatzstückes, ist der Sensorbereich zu dem ihm umgebenden Innenmantel des Rohres beabstandet und ermöglicht eine klare Unterscheidung zwischen der axialen Kraft in Rohr-längsrichtung und quer zur Rohrlängsrichtung oder um die Rohrlängsrichtung wirkenden Drehmomenten. Lediglich das Ende des Rohransatzstückes wird fest mit dem Rohr verbunden, so dass das Ende des Rohres, welches das Ende des Rohransatzstückes zum Adapteranschluss hin im Sensorbereich über-ragt, kraftmäßig vom Sensorbereich und vom

Adapteranschluss entkoppelt ist. Dabei ergibt sich ein relativ leichter, einfacher und kostengünstiger Aufbau mit klaren Kraftübertragungsmechanismen und entsprechender einfacherer reproduzierbarer Messbarkeit.

Für die einstückige Ausbildung von Rohransatzstück und Rohr wird keine gesonderte Klebeverbindung benötigt. Die zum Schutz der DMS-Messstellen bzw. des Sensorbereiches notwendige Abdeckung erfolgt bei der einstückigen Ausbildung durch eine zusätzlich am Rohr befestigte Abdeckung, die aber wegen der kraftmäßigen Entkopplung zum Sensorbereich und Adapteranschluss relativ unproblematisch anzubringen ist.

Je nach Anwendungsfall lassen sich mit der vorliegenden Sensorvorrichtung die Axialkraft in Rohrlängsrichtung und/oder Drehmomente quer zur Rohrlängsrichtung oder auch um die Rohrlängsrichtung messen. Selbstverständlich können mit geeigneten Sensoren auch Kräfte senkrecht zur Rohrlängsrichtung gemessen werden.

Gemäß einer bevorzugten Ausführungsform der Erfindung ist das Rohr bzw. die Abdeckung gegenüber dem Adapteranschluss durch eine elastische Dichtung abgedichtet. Eine elastische Dichtung verhindert das Eindringen von Wasser ohne die kraftmäßige Entkopplung gegenüber dem Adapteranschluss und dem Sensorbereich aufzuheben.

Der Justieradapter und Adapteranschluss sind über mindestens drei Justierschrauben gegeneinander fixiert, wobei die Zahl der Messstellen im Sensorbereich mindestens der Zahl der Justierschrauben entspricht.

Gemäß einer weiteren bevorzugten Ausführungsform der Erfindung ist der Justieradapter in Form eines umgekehrten Pyramidenstumpfes ausgebildet und weist vier zur Längsachse schräg verlaufende plane Justierflächen auf, die mit in dem Adapteranschluss angeordneten vier Justierschrauben zusammenwirken, wobei der Pyramidenstumpf in einen kugelabschnittsförmigen Sockel übergeht, der mit einer entsprechenden ringförmigen Anlagefläche des Adapteranschlusses korrespondiert. Dabei sind acht DMS-Messstellen auf dem Umfang des Sensorbereichs angeordnet. Die acht DMS-Messstellen sind in einer radialen Ebene gegenüber vier um 90 Grad versetzten Justierschrauben um jeweils 22,5 Grad versetzt angeordnet.

Eine Anzahl von DMS-Messstellen, die der doppelten Anzahl von Justierschrauben entspricht, hat sich dabei besonders bewährt. Grundsätzlich können aus den acht Messstellen mindestens quer zur Rohrlängsrichtung auftretende Drehmomente und eine Axialkraft in Rohrlängsrichtung ermittelt werden. Da das auftretende Drehmoment und die Axialkraft in der Regel aber Signalgrößen von unterschiedlicher Größenordnung entsprechen, hat es sich bewährt, zusätzliche DMS-Messstellen auf dem Umfang des Sensorbereiches zur getrennten Ermittlung der axialen Kraft anzuordnen. Dabei können den beispielsweise vier Justierschrauben zugeordnet, vier zusätzliche DMS-Messstellen in einer radialen Ebene angeordnet sein.

Nach einer weiteren bevorzugten Ausführungsform der Erfindung weist jede Messstelle zwei Dehnmessstreifen auf, die in Rohrlängsrichtung nebeneinander mit um 90 Grad zueinander versetzter Messgitterausrichtung angeordnet sind.

Gemäß einer weiteren bevorzugten Ausführungsform der Erfindung ist der Adapteranschluss mit dem Rohransatzstück einstückig aus Titan ausgebildet. Dies hat den Vorteil, dass der Adapteranschluss bei geringem Gewicht und einer relativ dünnen Rohrwandung im Sensorbereich eine relativ hohe Festigkeit aufweist.

Die Sensoreinrichtung kann beispielsweise in einem künstlichen Bein oder einem Roboterarm einer Handhabungsvorrichtung verwendet werden.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:

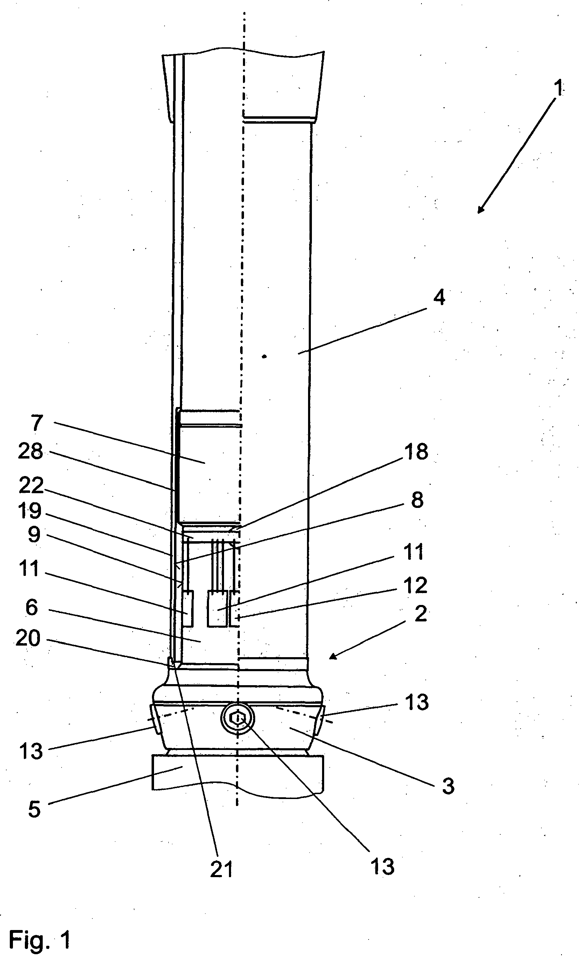

- Figur 1:: eine Seitenansicht einer Sensoreinrichtung mit in einem Rohr angeordnetem Messelement, wobei das Rohr mit einer elastischen Dichtung im Halbschnitt dargestellt ist,

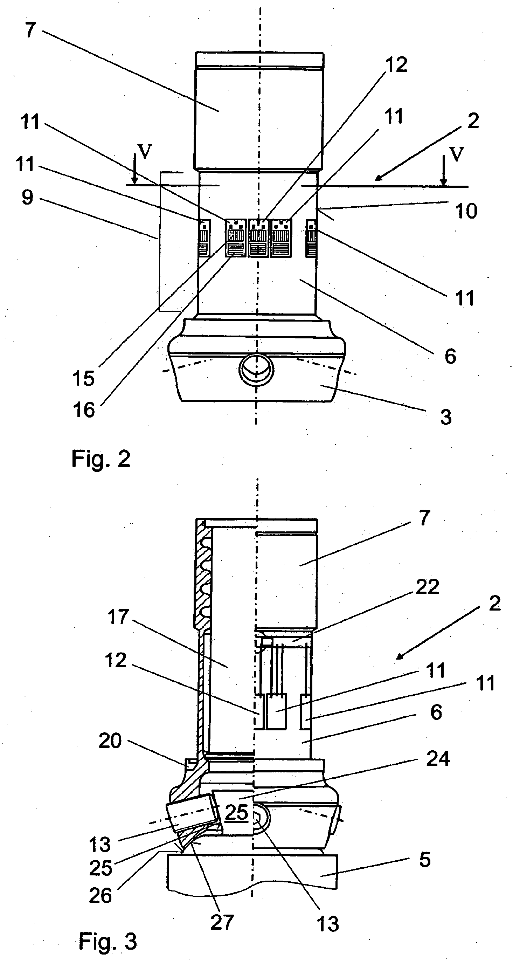

- Figur 2:: eine Vorderansicht des Messelementes von Figur 1,
- Figur 3:: eine Seitenansicht im Halbschnitt des Messelementes von Figur 1 mit angekoppeltem Justieradapter,

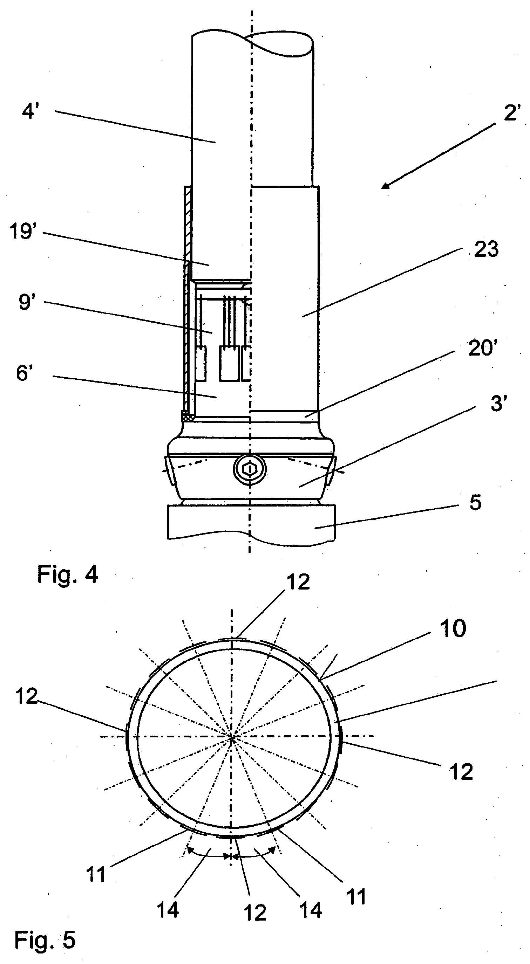

- Figur 4:: eine Seitenansicht eines an ein Rohr angeformten Messelementes mit angekoppeltem Justieradapter und Abdeckung des Sensorbereiches im Halbschnitt und
- Figur 5:: eine Draufsicht auf den Sensorbereich von Figur 2 entlang der Linie V-V geschnitten.

### Beschreibung der Ausführungsbeispiele

Eine Sensoreinrichtung 1 besteht im Wesentlichen aus einem Messelement 2 mit einem Adapteranschluss 3, über den ein Rohr 4 an einen Justieradapter 5 ankoppelbar ist.

Das Messelement 2 besteht aus einem Rohransatzstück 6, das an seinem ersten Ende den Adapteranschluss 3 aufweist. Mit seinem dem Adapteranschluss 3 abgewandten freien Ende 7 ist das Rohransatzstück 6 in das Rohr 4 einsetzbar und mit dessen Rohrinnenwandung 8 unter Einsatz eines Klebstoffes 28 verklebbar. Zwischen dem freien Ende 7 und dem Adapteranschluss 3 befindet sich ein Sensorbereich 9, der einen geringeren Außendurchmesser 10 als das freie Ende 7 aufweist. Auf dem Umfang des Sensorbereiches 9 sind im Ausführungsbeispiel acht DMS-Messstellen 11 zur Drehmomentenbestimmung und vier DMS-Messstellen 12 zur Axialkraftbestimmung angeordnet. Die acht DMS-Messstellen 11 zur Drehmomentenbestimmung sind in einer radialen Ebene gegenüber vier um 90° versetzten Justierschrauben 13 im Adapteranschluss 3 um jeweils einen Winkel 14 von 22,5° versetzt angeordnet. Die DMS-Messstellen 12 zur Axialkraftbestimmung sind in der radialen Ebene den Justierschrauben 13 zugeordnet und liegen damit zwischen zwei DMS-Messstellen 11 zur Drehmomentenbestimmung. Jede der Messstellen 11, 12 weist jeweils zwei Dehnmessstreifen 15, 16 auf, die in Rohrlängsrichtung nebeneinander mit um 90° zueinander versetzter Messgitterausrichtung angeordnet sind. Die Dehnmessstreifen 15, 16 sind auf dem Umfang des Sensorbereiches 9 in bekannter Weise aufgeklebt. Das Messelement 2 ist vorteilhaft aus Titan ausgebildet.

In das freie Lumen des Rohransatzstückes 6 ist eine zylinderförmige Messelektronik 17 einsetzbar, von der über Querbohrungen 18 im Sensorbereich 9 Flachkabel 22 nach außen führbar sind, die mit Anschlusspunkten der Messstellen 11, 12 verbunden werden.

Das Messelement 2 wird mit dem freien Ende 7 seines Rohransatzstückes 6 in das freie Lumen des Rohres 4 eingesetzt und mit dessen Rohrinnenwandung 8 verklebt. Das Rohr 4 überragt mit seinem dem Adapteranschluss 3 zugewandten Ende 19 den Sensorbereich 9 und bildet zu dem Adapteranschluss 3 einen Abstand, wobei das Ende 19 des Rohres 4 gegenüber dem Adapteranschluss 3 durch eine elastische Dichtung 20 abgedichtet wird. Damit ist das Rohr 4 kraftmäßig mit dem Messelement 2 nur über das freie Ende 7 des Rohransatzstückes 6 verbunden. Durch den Abstand, den das Ende 19 mit seiner Stirnfläche 21 gegenüber den Adapteranschluss 3 und mit seinem Innendurchmesser bzw. seiner Rohrinnenwandung 8 vom Sensorbereich 9 aufweist, ist das Rohrende 19 vom Adapteranschluss 3 und vom Sensorbereich 9 kraftmäßig entkoppelt.

Nach einer anderen Ausführungsform, die nicht zu der Erfindung gehört, ist das Rohransatzstück 6' des Messelementes 2' an das dem Adapteranschluss 3' zugewandte Ende 19' des Rohres 4' angeformt. Dabei wird der Sensorbereich 9' von einer am Rohr befestigten Abdeckung 23 abgedeckt, die kraftmäßig vom Sensorbereich 9' und vom Adapteranschluss 3' entkoppelt ist. Zur Abdichtung ist dabei zwischen der Abdeckung 23 und dem Adapteranschluss 3' eine elastische Dichtung 20' angeordnet.

Der Justieradapter 5 weist in an sich bekannter Weise die Form eines umgekehrten Pyramidenstumpfes 24 mit vier schräg verlaufenden planen Justierflächen 25 auf, die mit den Justierschrauben 13 zusammenwirken, wobei der Pyramidenstumpf 24 in einen kugelabschnittsförmigen Sockel 26 übergeht, der mit einer entsprechenden ringförmigen Anlagefläche 27 des Adapteranschlusses 3 korrespondiert.

## Patentansprüche

1. Sensoreinrichtung (1) zur Messung einer axialen Kraft in Rohrlängsrichtung eines Rohres (4) und mindestens eines Drehmomentes mit einem Messelement (2), das einen über ein Rohransatzstück (6) mit dem Rohr (4) verbundenen Adapteranschluss (3, 3') aufweist, über den das Rohr (4) an einen Justieradapter (5) fest ankoppelbar ist, wobei die axiale Kraft und das Drehmoment über das Messelement in den Justieradapter (5) einleitbar und mittels Dehnmessstreifen (15, 16) messbar sind, die in einem Sensorbereich (9) zwischen dem Adapteranschluss (3) und einem dem Adapteranschluss (3) abgewandten Ende (7) des Rohransatzstückes (6) an DMS-Messstellen (11, 12) angeordnet sind, wobei das Rohransatzstück (6) mit dem Sensorbereich (9) rohrförmig ausgebildet ist, der Sensorbereich (9) sich von dem Adapteranschluss bis zu dem Ende (7) des Rohransatzstückes (6) erstreckt und einen geringeren Außendurchmesser (10) als das Ende (7) des Rohransatzstückes (6) aufweist, wobei das Rohr (4) mit seinem dem Adapteranschluss (3) zugewandten, das Ende (7) des in das Rohr (4) einsetzbaren Rohransatzstückes (6) zum Adapteranschluss (3) hin überragenden Ende (7) kraftmäßig vom Sensorbereich (9) und vom Adapteranschluss (3) entkoppelt ist, **dadurch gekennzeichnet, dass** auf dem Umfang des Sensorbereiches (9) mindestens drei DMS-Stellen (11, 12) angeordnet sind und dass zur von der Drehmomentmessung getrennten Ermittlung der axialen Kraft zusätzliche DMS-Stellen (11, 12) auf dem Umfang des Sensorbereiches (9, 9') angeordnet sind.

2. Sensoreinrichtung nach Anspruch 1, **dadurch gekennzeichnet, dass** das Rohr (4, 4') oder die Abdeckung (23) gegenüber dem Adapteranschluss (3, 3') durch eine elastische Dichtung (20, 20') abgedichtet ist.

3. Sensoreinrichtung nach Anspruch 1 oder 2, **dadurch gekennzeichnet, dass** der Justieradapter (5) und der Adapteranschluss (3, 3') über mindestens drei Justierschrauben (13) miteinander zusammenwirken und dass die Zahl der Messstellen (15, 16) mindestens der Zahl der Justierschrauben (13) entspricht.

4. Sensoreinrichtung nach einem der Ansprüche 1 bis 3, **dadurch gekennzeichnet, dass** der Justieradapter (5) in Form eines umgekehrten Pyramidenstumpfes (24) ausgebildet ist und vier schräg verlaufende plane Justierflächen (25) aufweist, die mit in dem Adapteranschluss (3, 3') angeordneten vier Justierschrauben (13) zusammenwirken können, wobei der Pyramidenstumpf (24) in einen kugelabschnittsförmigen Sockel (26) übergeht, der mit einer entsprechenden ringförmigen Anlagefläche (27) des Adapteranschlusses (3, 3') korrespondieren kann.

5. Sensoreinrichtung nach einem der Ansprüche 1 bis 4, **dadurch gekennzeichnet, dass** acht DMS-Messstellen (11, 12) auf dem Umfang des Sensorbereiches (9, 9') angeordnet sind.

6. Sensoreinrichtung nach Anspruch 5, **dadurch gekennzeichnet, dass** die acht DMS-Messstellen (11, 12) in einer radialen Ebene gegenüber vier um 90° versetzten Justierschrauben (13) um jeweils 22,5° versetzt angeordnet sind.

7. Sensoreinrichtung nach Anspruch 6, **dadurch gekennzeichnet, dass** vier zusätzliche DMS-Messstellen (11, 12) in einer radialen Ebene den Justierschrauben (13) zugeordnet angeordnet sind.

8. Sensoreinrichtung nach einem der Ansprüche 1 bis 7, **dadurch gekennzeichnet, dass** jede DMS-Messstelle (11, 12) mindestens einen jeweils einer Brückenschaltung zugeordneten Dehnmessstreifen (15, 16) aufweist.

9. Sensoreinrichtung nach Anspruch 8, **dadurch gekennzeichnet, dass** jede Messstelle (11, 12) zwei Dehnmessstreifen (15, 16) aufweist, die in Rohrlängsrichtung nebeneinander mit um 90° zueinander versetzter Messgitterausrichtung, angeordnet sind.

10. Sensoreinrichtung nach einem der Ansprüche 1 bis 9, **dadurch gekennzeichnet, dass** der Adapteranschluss (3, 3') mit dem Rohransatzstück (6, 6') einstückig aus Titan ausgebildet ist.

11. Sensoreinrichtung nach einem der Ansprüche 1 bis 10, **dadurch gekennzeichnet, dass** die Sensoreinrichtung (1) in einem künstlichen Glied verwendet wird.

12. Sensoreinrichtung nach Anspruch 11, **dadurch gekennzeichnet, dass** die Sensoreinrichtung (1) in einem Bein verwendet wird.

13. Sensoreinrichtung nach einem der Ansprüche 1 bis 10, **dadurch gekennzeichnet, dass** die Sensoreinrichtung (1) in einem Roboterarm einer Handhabungsvorrichtung verwendet wird.

## Claims

1. A sensor device (1) for measuring an axial force in the longitudinal pipe direction of a pipe (4) and at least one torque with a measuring element (2) which has an adaptor connection (3, 3'), which is connected to the pipe (4) by means of a pipe extension piece (6) and which can be used to firmly couple the pipe (4) to an alignment adaptor (5), wherein the axial force and the torque can be introduced into the alignment adaptor (5) by means of the measuring element (2) and can be measured by means of strain gauges (15, 16) which are arranged at DMS measuring points (11, 12) in a sensor region (9) between the adaptor connection (3) and an end (7) of the pipe extension piece (6) which is remote from the adaptor connection (3), wherein the pipe extension piece (6) with the sensor region (9) is of tubular design, the sensor region (9) extends from the adaptor connection (3) to the end (7) of the pipe extension piece (6) and has a smaller external diameter (10) than the end (7) of the pipe extension piece (6), wherein the pipe (4) has its end (7) which faces the adaptor connection (3) and which overtops the end (7) of the pipe extension piece (6), which can be inserted into the pipe (4), toward the adaptor connection (3) decoupled in terms of force from the sensor region (9) and from the adaptor connection (3), **characterized in that** at least three DMS measuring points (11, 12) are arranged on the perimeter of the sensor region (9) and that additional DMS measuring points (11, 12) are arranged on the perimeter of the sensor region (9, 9') for the purpose of ascertainment of the axial force separate from the torque measurement.

2. The sensor device as claimed in claim 1, **characterized in that** the pipe (4, 4') or the cover (23) is sealed from the adaptor connection (3, 3') by an elastic seal (20, 20').

3. The sensor device as claimed in claim 1 or 2, **characterized in that** the alignment adaptor (5) and the adaptor connection (3, 3') interact with one another via at least three alignment screws (13), and **in that** the number of measuring points (15, 16) corresponds at least to the number of alignment screws (13).

4. The sensor device as claimed in one of claims 1 to 3, **characterized in that** the alignment adaptor (5) is in the form of an inverted pyramid frustum (24) and has four obliquely running planar alignment faces (25) which can interact with four alignment screws (13) arranged in the adaptor connection (3, 3'), wherein the pyramid frustum (24) merges into a sphere-section-shaped base (26) which can correspond with a corresponding annular contact face (27) of the adaptor connection (3, 3').

5. The sensor device as claimed in one of claims 1 to 4, **characterized in that** eight DMS measuring points (11, 12) are arranged on the perimeter of the sensor region (9, 9').

6. The sensor device as claimed in claim 5, **characterized in that** the eight DMS measuring points (11, 12) are arranged in a radial plane with a respective 22.5° offset from four alignment screws (13) having a 90° offset.

7. The sensor device as claimed in claim 6, **characterized in that** four additional DMS measuring points (11, 12) are arranged in a radial plane in association with the alignment screws (13).

8. The sensor device as claimed in one of claims 1 to 7, **characterized in that** each DMS measuring point (11, 12) has at least one strain gauge (15, 16) associated with a respective bridge circuit.

9. The sensor device as claimed in claim 8, **characterized in that** each measuring point (11, 12) has two strain gauges (15, 16) which are arranged next to one another in the longitudinal pipe direction with a measuring grid orientation having a 90° offset from one another.

10. The sensor device as claimed in one of claims 1 to 9, **characterized in that** the adaptor connection (3, 3') is produced from titanium in one piece with the pipe extension piece (6, 6').

11. The sensor device as claimed in one of claims 1 to 10, **characterized in that** the sensor device (1) is used in an artificial limb.

12. The sensor device as claimed in claim 11, **characterized in that** the sensor device (1) is used in a leg.

13. The sensor as claimed in one of claims 1 to 10, **characterized in that** the sensor device (1) is used in a robot arm of a handling apparatus.

## Revendications

1. Dispositif de détection (1) pour mesurer une force axiale en direction longitudinale d'un tube (4) et au moins un couple au moyen d'un élément de mesure (2) qui présente un raccord d'adaptation (3, 3') relié au tube (4) via un embout de tube (6), par l'intermédiaire duquel le tube (4) peut être raccordé de manière solidaire à un adaptateur de réglage (5), dans lequel la force axiale et le couple peuvent être introduits, via l'élément de mesure, dans l'adaptateur de réglage et peuvent être mesurés au moyen de jauges de contrainte (DMS) (15, 16) qui sont agencées à des points de mesure de DMS dans une zone de détection (9) entre le raccord d'adaptation (3) et une extrémité (7), détournée du raccord d'adaptation (3) de l'embout de tube (6), l'embout de tube (6) étant réalisé de forme tubulaire avec la zone de détection (9), la zone de détection (9) s'étendant depuis le raccord d'adaptation jusqu'à l'extrémité (7) de l'embout de tube (6) et présentant un diamètre extérieur (10) inférieur à l'extrémité (7) de l'embout de tube (6), le tube (4) étant, par son extrémité (7) tournée vers le raccord d'adaptation (3) qui dépasse l'extrémité de l'embout de tube (6), découplé, par application de force, vis-à-vis de la zone de détection (9) et du raccord d'adaptation (3), **caractérisé en ce que** sur la périphérie de la zone de détection (9) sont agencés au moins trois points de mesure de DMS (11, 12), et **en ce que**, pour déterminer la fore axiale, séparément de la mesure de couple, des points de mesure de DMS (11, 12) supplémentaires sont agencés sur la périphérie de la zone de détection (9, 9').

2. Dispositif de détection selon la revendication 1, **caractérisé en ce que** le tube (4, 4') ou le recouvrement (23) est étanché vis-à-vis du raccord d'adaptation (3, 3') par un joint élastique (20, 20').

3. Dispositif de détection selon la revendication 1 ou 2, **caractérisé en ce que** l'adaptateur de réglage (5) et le raccord d'adaptation (3, 3') coopèrent l'un avec l'autre via au moins trois vis de réglage (13), et **en ce que** le nombre de points de mesure (15, 16) correspond au moins au nombre des vis de réglage (13).

4. Dispositif de détection selon l'une des revendications 1 à 3, **caractérisé en ce que** l'adaptateur de réglage (5) est réalisé sous la forme d'un tronc de pyramide (24) inversé et présente quatre surfaces de réglage (25) planes s'étendant en oblique qui peuvent coopérer avec quatre vis de réglage (13) agencées dans le raccord d'adaptation (3, 3'), le tronc de pyramide (24) se transformant en un socle (26) en forme de tronçon sphérique qui peut correspondre avec une surface d'appui (27) annulaire correspondante du raccord d'adaptation (3, 3').

5. Dispositif de détection selon l'une des revendications 1 à 4, **caractérisé en ce que** huit points de mesure de DMS (11, 12) sont agencés sur la périphérie de la zone de détection (9, 9').

6. Dispositif de détection selon la revendication 5, **caractérisé en ce que** les huit points de mesure de DMS (11, 12) sont agencés dans un plan radial, chacun en décalage de 22,5° par rapport aux vis de réglages (13) décalées de 90°.

7. Dispositif de détection selon la revendication 6, **caractérisé en ce que** quatre points de mesure de DMS (11, 12) supplémentaires sont associés aux vis de réglage (13) dans un plan radial.

8. Dispositif de détection selon l'une des revendications 1 à 7, **caractérisé en ce que** chaque point de mesure de DMS (11, 12) présente au moins une jauge de contrainte associée respectivement à un circuit en pont.

9. Dispositif de détection selon la revendication 8, **caractérisé en ce que** chaque point de mesure (11, 12) présente deux jauges de contrainte (15, 16) qui sont agencées en direction longitudinale de tube l'une à côté de l'autre avec une orientation de grille de mesure décalée de 90° l'une par rapport à l'autre.

10. Dispositif de détection selon l'une des revendications 1 à 9, **caractérisé en ce que** le raccord d'adaptation (3, 3') est réalisé en titane d'un seul tenant avec l'embout de tube (6, 6').

11. Dispositif de détection selon l'une des revendications 1 à 10, **caractérisé en ce que** le dispositif de détection (1) est utilisé dans un membre artificiel.

12. Dispositif de détection selon la revendication 11, **caractérisé en ce que** le dispositif de détection (1) est utilisé dans une jambe.

13. Dispositif de détection selon l'une des revendications 1 à 10, **caractérisé en ce que** le dispositif de détection (1) est utilisé dans un bras de robot d'un dispositif de manipulation.
